# EUROPEAN PATENT APPLICATION

(11) **EP 2 940 462 A1**
(43) Date of publication of application: **04.11.2015**
(21) Application number: 13867716.6
(22) Date of filing: 26.12.2013
(51) Int. Cl.: G01N 27/00, G01N 27/12, C01B 31/02

(54) **GAS SENSOR AND GAS SENSOR STRUCTURAL BODY**

(30) Priority: 28.12.2012 JP 2012286557
(71) Applicant: The University of Tokyo, Tokyo 113-8654 (JP); Omron Corporation, Kyoto-shi, Kyoto 600-8530 (JP)
(72) Inventor: SHIMOYAMA, Isao, Tokyo 113-8654 (JP); MATSUMOTO, Kiyoshi, Tokyo 113-8654 (JP); TAKEI, Yusuke, Tokyo 113-8654 (JP); INABA, Akira, Tokyo 113-8654 (JP); YOO, Kwanghyun, Tokyo 113-8654 (JP); HONDA, Masahito, Kyoto-shi, Kyoto 600-8530 (JP); IMAMOTO, Hiroshi, Kyoto-shi, Kyoto 600-8530 (JP)
(74) Representative: Fuchs Patentanwälte Partnerschaft mbB
(86) International application number: PCT/JP2013/084795
(87) International publication number: WO 2014/104156

(57) **Abstract**

To propose a gas sensor and a gas sensor structural body that can improve detection sensitivity of gas more than in the conventional gas sensors with a simple configuration. A graphene (8) between a source electrode (3) and a drain electrode (4) is provided in an ion liquid (L), whereby a state change of charges in the ion liquid (L) caused by absorption of gas is directly reflected on a source-drain current (I_{sd}) that flows in the graphene (8). Therefore, it is possible to improve detection sensitivity of the gas more than in the conventional gas sensors. The graphene (8) only has to be provided to be disposed in the ion liquid (L). Therefore, unlike in the conventional gas sensors, a configuration in which carbon nanotubes are surface-chemically modified by a plurality of polymers is unnecessary. Therefore, it is possible to simplify a configuration.

## Description

### FIELD

The present invention relates to a gas sensor and a gas sensor structural body, and is suitable for detection of gases such as CO₂ and NH₃.

### BACKGROUND

In recent years, there have been conducted researches of gas sensors that can detect various gases such as CO₂ and NH₃. Among the gas sensors, for example, a gas sensor including carbon nanotubes (CNTs) attracts particular attention from the viewpoints of detection sensitivity of gas, a reduction in size, and energy saving (see, for example, Patent Literature 1 and Non Patent Literature 1). Practically, for example, in order to detect a detection target CO₂, such a gas sensor including the carbon nanotubes has a configuration in which the carbon nanotubes provided between a source electrode and a drain electrode are surface-chemically modified by two kinds of polymers. In the gas sensor, the carbon nanotubes are disposed on a silicon back gate via a silicon oxide film. The gas sensor is configured such that a gate voltage is applied to the silicon back gate.

### Citation List

### Patent Literature

Patent Literature 1: National Publication of International Patent Application No. 2007-505323

### Non Patent Literature

Non Patent Literature 1: A. Star, T. R. Han, V. Joshi, J. C. P. Gabriel, G. Gruner, "Nanoelectronic Carbon Dioxide Sensors", Advanced Materials, Vol. 16, No. 22, 2004.

### SUMMARY

### Technical Problem

However, in order to make it possible to detect CO₂ using the carbon nanotubes, the gas sensor having such a configuration needs to perform surface chemical modification of the carbon nanotubes with the two kinds of polymers. Therefore, there is a problem in that the configuration is complicated. Such a gas sensor can detect a detection target gas. However, there is a further demand for improvement of detection sensitivity such that even a very small amount of gas can be detected.

Consequently, the present invention has been devised in view of the above points. An object of the present invention is to propose a gas sensor and a gas sensor structural body that can improve gas detection sensitivity more than the conventional gas sensors, with a simple configuration.

### Solution to Problem

An aspect of the present invention is a gas sensor that detects a gas as a target for detection. The gas sensor includes: a carbon structural body provided between a source electrode and a drain electrode on a substrate, flat and formed of a single-layer structure having thickness equivalent to one carbon atom or formed of a plural-layer structure; and a gas absorbent disposed to cover the carbon structural body. The gas sensor detects the gas on the basis of a change in a source-drain current caused in the carbon structural body by absorption of the gas via the gas absorbent.

Another aspect of the present invention is a gas sensor structural body having a configuration in which a plurality of gas sensors are disposed. The gas sensors are the gas sensor as set forth in any one of claims 1 to 9.

### Advantageous Effects of Invention

According to the present invention, a state change of charges in the gas absorbent caused by the absorption of the gas is directly reflected on the source-drain current that flows in the carbon structural body. Therefore, it is possible to improve detection sensitivity of the gas more than in the conventional gas sensors. Further, unlike in the conventional gas sensors, it is unnecessary to apply surface chemical modification to carbon nanotubes themselves. The gas absorbent only has to be provided in contact with the carbon structural body. Therefore, it is possible to simplify a configuration.

### BRIEF DESCRIPTION OF THE DRAWINGS

FIG. 1 is a perspective view showing the configuration of a gas sensor according to the present invention.
FIG. 2 is a sectional view showing the sectional configuration of the gas sensor in FIG. 1.
FIG. 3 is a schematic diagram illustrating an electric double layer.
FIG. 4A is a schematic diagram illustrating the electric double layer at the time when a gas absorbent absorbs NH₃; and FIG. 4B is a schematic diagram illustrating the electric double layer at the time when the gas absorbent absorbs CO₂.
FIG. 5 is a graph showing changes in a source-drain current I_{sd} and a gate voltage V_{g}.
FIG. 6A to FIG. 6F are schematic diagrams for explanation (1) of a manufacturing method for the gas sensor.
FIG. 7A to FIG. 7D are schematic diagrams for explanation (2) of the manufacturing method for the gas sensor.
FIG. 8 is a photograph showing the configuration of an actually manufactured gas sensor and the detailed configuration of a graphene.
FIG. 9 is a graph showing an analysis result of a Raman shift of the graphene.
FIG. 10 is a photograph showing the configuration of a gas sensor used for a verification test.
FIG. 11 is a graph showing a relation between a gate current I_{g} and the gate voltage V_{g} obtained by the gas sensor under the air atmosphere.
FIG. 12 is a graph showing a relation between the source-drain current I_{sd} and the gate voltage V_{g} obtained by the gas sensor under the air atmosphere.
FIG. 13 is a schematic diagram showing the overall configuration of an experimental apparatus.
FIG. 14A is a graph showing a relation between the source-drain current I_{sd} and the gate voltage V_{g} at the time when NH₃ concentration is changed; and FIG. 14B is a graph showing a relation between the source-drain current I_{sd} and the gate voltage V_{g} at the time when CO₂ concentration is changed.
FIG. 15A is a graph showing a relation between the source-drain current I_{sd} and the NH₃ concentration; and FIG. 15B is a graph showing a relation between the source-drain current I_{sd} and the CO₂ concentration.
FIG. 16A is a graph showing a response time of the source-drain current I_{sd} to NH₃; and FIG. 16B is a graph showing a response time of the source-drain current I_{sd} at the time when the distance between the graphene and an ion liquid is changed.
FIG. 17 is a perspective view showing the configuration of a gas sensor (1) according to another embodiment.
FIG. 18 is a sectional view showing the sectional configuration of the gas sensor in FIG. 17.
FIG. 19 is a sectional view showing the sectional configuration of a gas sensor (2) according to another embodiment.
FIG. 20 is a perspective view showing the configuration of a gas sensor (3) according to another embodiment.

### DETAILED DESCRIPTION

Embodiments of the present invention are explained in detail below with reference to the drawings.

### (1) Overview of a gas sensor of the present invention

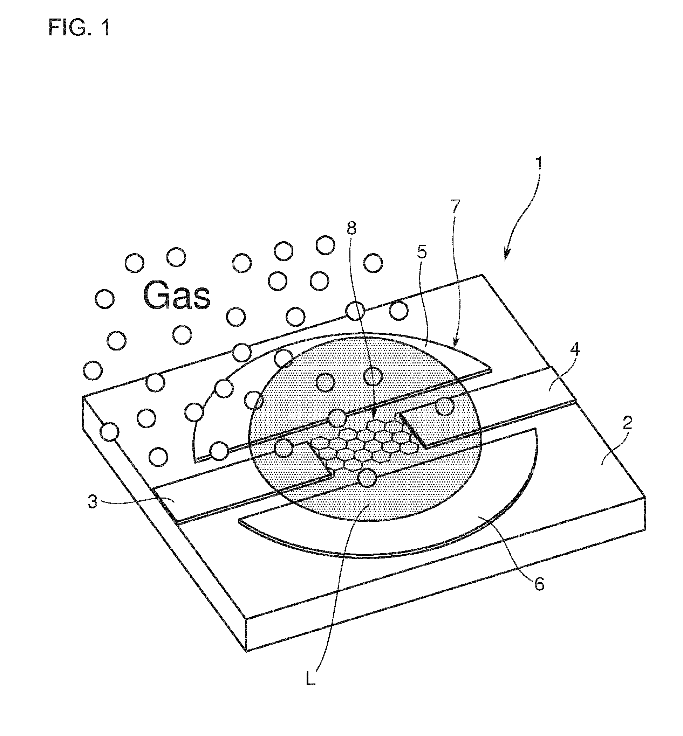

In FIG. 1, reference numeral 1 denotes a gas sensor according to the present invention. The gas sensor 1 is configured to be capable of detecting gas such as CO₂ or NH₃ as a detection target. The gas sensor 1 has a characteristic in that a film-like graphene 8 is provided as a flat carbon structural body between a source electrode 3 and a drain electrode 4. Practically, the gas sensor 1 includes the source electrode 3 and the drain electrode 4 having a belt shape on the substrate 2 formed in a tabular shape. The flat film-like graphene 8 is provided in a channel region between the source electrode 3 and the drain electrode 4. The source electrode 3 and the drain electrode 4 are formed of, for example, a Cr/Au member or a Ti/Au member and disposed substantially linearly with longitudinal directions thereof aligned. A gap of approximately 10 [µm] is formed between an end of the source electrode 3 and an end of the drain electrode 4 opposed to each other.

The graphene 8 has film thickness of, for example, 0.3 to 3 [nm] and is formed in a quadrilateral shape such as a rectangular shape or a square shape. One end side of the graphene 8 is electrically connected to the end of the source electrode 3. The other end side of the graphene 8 is electrically connected to the end of the drain electrode 4. The graphene 8 is disposed to electrically connect the source electrode 3 and the drain electrode 4. Here, the graphene 8 refers to a graphene formed of a single-layer structure in which six-membered ring structures formed by combination of carbon atoms are regularly arrayed in a two-dimensional plane shape and one mesh-like plane layer having thickness equivalent to one atom is disposed or a plural-layer structure in which ten or less mesh-like plane layers are stacked. Note that the graphene formed of the plural-layer structure is desirably a stacked structure of two or three layers. In the present invention, since the flat film-like graphene 8 is used, the entire surface of the graphene 8 can be formed in surface-contact on the substrate 2. The entire surface of the graphene 8 can be exposed in an ion liquid L dropped on the substrate 2. Since the film thickness is small, a reduction in the thickness of the entire gas sensor 1 can also be attained.

Note that, in the embodiment explained above, the graphene 8 is applied as the flat carbon structural body. However, the present invention is not limited to this. Other various flat carbon structural bodies such as a flat film-like graphene stacked body in which eleven or more mesh-like plane layers are stacked and a tabular graphite in which one hundred or more mesh-like plane layers are stacked may be applied.

A gate electrode 7 formed of, for example, a Cr/Au member or a Ti/Au member is provided on the substrate 2. The ion liquid L is placed in contact with the gate electrode 7 to enable the graphene 8 to be contained the ion liquid L. The gate electrode 7 includes a first gate electrode section 5 and a second gate electrode section 6 formed in the same shape and the same size. The graphene 8, the source electrode 3, and the drain electrode 4 can be disposed in a gap between the first gate electrode section 5 and the second gate electrode section 6. Specifically, in the case of this embodiment, the first gate electrode section 5 and the second gate electrode section 6 are formed in a semicircular shape. A linear section of the first gate electrode section 5 and a linear section of the second gate electrode section 6 are disposed in parallel a predetermined gap apart from each other. In the gap of these linear sections, the graphene 8, the source electrode 3, and the drain electrode 4 are linearly disposed with longitudinal directions thereof aligned.

The ion liquid L is placed in a hemispherical shape over the first gate electrode section 5, the second gate electrode section 6, the source electrode 3, and the drain electrode 4 to cover the entire graphene 8 and configured to be capable of forming an electric double layer functioning as a gate insulating layer in a part of the ion liquid L. The hemispherical liquid surface of the ion liquid L is exposed to the outside air. The ion liquid L is configured to contain the graphene 8 present in the center of the first gate electrode section 5, the second gate electrode section 6, the source electrode 3, and the drain electrode 4. The ion liquid L functioning as a gas absorbent includes, for example, besides [EMIM][BF₄] (1-ethyl-3-methyl imidazolium tetrafluoroborate) [BMIM][BF₄] (1-butyl-3-methyl imidazolium tetrafluoroborate), [BMIM][PF₆] (1-butyl-3-methyl imidazolium hexafluorophosphate), and [OMIM][Br] (1-n-octyl-3-methyl imidazolium bromide), [Hmpy][Tf₂N], [HMIM] [Tf₂N], [BMIM][Tf₂N], [C₆H₄F₉mim][Tf₂N], [AMIM][BF₄], [Pabim][BF₄], [Am-im][DCA], [Am-im][BF₄], [BMIM][BF₄]+PVDF, [C₃NH₂mim][CF₆SO₃]+PTFE, [C₃NH₂mim][Tf₂N]+PTFE, [H₂NC₃H₆mim][Tf₂N]+cross-linked Nylon66, P [VBBI] [BF₄], P[MABI][BF₄], P[VBBI][Tf₂N], P[VBTMA][BF₄], and P[MATMA][BF₄]. According to a type of gas as a target for detection, ion liquid capable of absorbing the gas can be selected as appropriate.

For example, when the gas sensor 1 capable of detecting CO₂ is provided, [EMIM][BF₄], [EMIM][BF₄], [BMIM][BF₄], [BMIM][PF₆], [Hmpy][Tf₂N], [HMIM][Tf₂N], [BMIM][Tf₂N], [C₆H₄F₉mim][Tf₂N], [AMIM][BF₄], [Pabim][BF₄], [Am-im][DCA], [Am-im][BF₄], [BMIM][BF₄]+PVDF, [C₃NH₂mim][CF₆SO₃]+PTFE, [C₃NH₂mim][Tf₂N]+PTFE, [H₂NC₃H₆mim][Tf₂N]+cross-linked Nylon66, P [VBBI] [BF₄], P[MABI][BF₄], P[VBBI][Tf₂N], P[VBTMA][BF₄], P[MATMA][BF₄], or the like capable of absorbing CO₂ is used as the ion liquid L. When the gas sensor 1 capable of detecting NH₃ is provided, [EMIM][BF₄] or the like capable of absorbing NH₃ is used as the ion liquid L.

Note that, for example, PEI (polyethyleneimine) may be added to the ion liquid L. In the ion liquid L added with the PEI, an amino group of the PEI can move charges to the graphene 8 and reduce a resistance value of the graphene 8. In the ion liquid L added with the PEI, when the ion liquid L absorbs gas, the PEI reacts with CO₂ or H₂O and the amino group of the PEI decreases. Therefore, in the gas sensor 1 that uses the ion liquid L added with the PEI, when the ion liquid L absorbs the outside air having a high content of CO₂, the amino group of the PEI in the ion liquid L decreases. As a result, the resistance value of the graphene 8 can increase. An electric state of the graphene 8 can change according to the content of CO₂ in the outside air.

Further, in the embodiment explained above, the ion liquid L is applied as the gas absorbent. However, the present invention is not limited to this. Other various liquid-like, gel-like, and cream-like gas absorbents such as hydroxide aqueous solutions of alkali metal and alkali earth metal may be applied. Note that, when the hydroxide aqueous solutions of alkali metal and alkali earth metal are used as the gas absorbent, CO₂ can be absorbed. Therefore, it is possible to realize a gas sensor that detects CO₂ as a detection target.

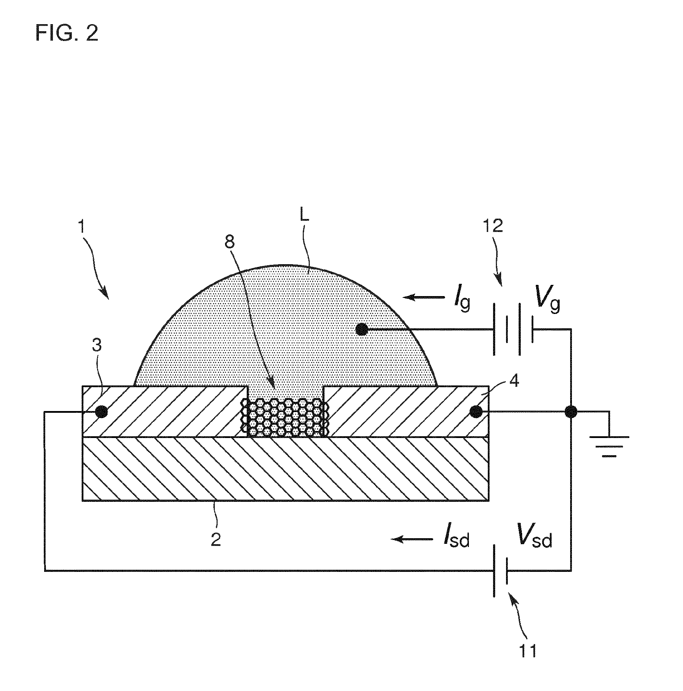

In the case of this embodiment, as shown in FIG. 2, one end side of the graphene 8 is covered by the source electrode 3 and the other end side of the graphene 8 is covered by the drain electrode 4. Consequently, both the ends are surely fixed to the substrate 2. When the ion liquid L is dropped, the graphene 8 does not come off the substrate 2. An electric connection state of the graphene 8 to the source electrode 3 and the drain electrode 4 can be maintained. Note that, actually, the graphene 8 is also formed right under the source electrode 3 and the drain electrode 4. However, in FIG. 2, for convenience of explanation, only the graphene 8 functioning as a channel between the source electrode 3 and the drain electrode 4 is shown.

The gas sensor 1 is configured such that a source-drain current I_{sd} is supplied from the source electrode 3 to the drain electrode 4 by a power supply 11 and a gate voltage V_{g} is applied to the first gate electrode section 5 and the second gate electrode section 6 by a power supply 12.

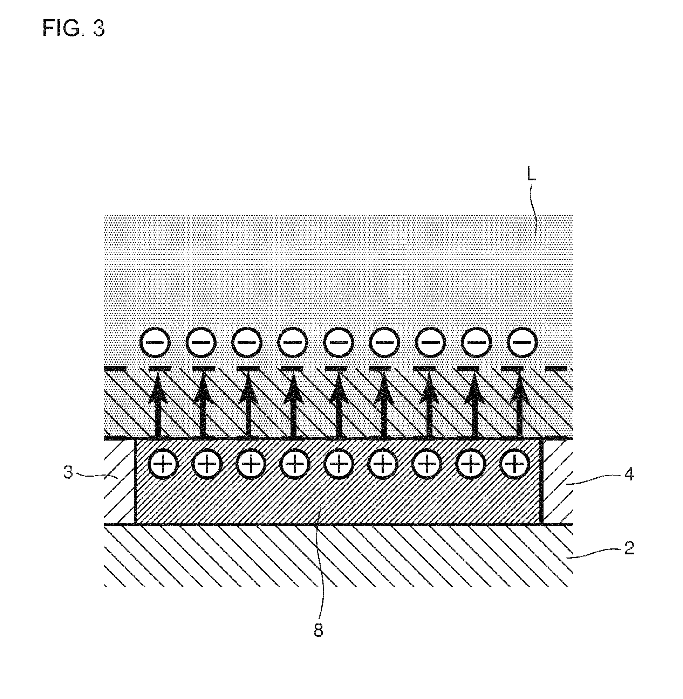

Consequently, in the gas sensor 1, when the gate voltage V_{g} is applied to the first gate electrode section 5 opposed to the graphene 8, a potential difference occurs in the ion liquid L. Charges are supplied to the graphene 8 in order to keep balance. Specifically, when a negative voltage is applied to the first gate electrode section 5, charges in the ion liquid L are polarized and negative charges gather on the surface of the graphene 8. Conversely, it is also possible to apply a positive voltage to the first gate electrode section 5. In that case, similarly, the charges in the ion liquid L are polarized. However, positive charges gather on the surface of the graphene 8. Consequently, for example, when the negative voltage is applied to the gate electrode 7, in the gas sensor 1, as shown in FIG. 3, the negative charges in the ion liquid L gather on the surface of the graphene 8, an electric double layer is formed in the ion liquid L, and the electric double layer can be a gate insulating layer.

That is, in the gas sensor 1, when the gate voltage V_{g} is applied to the first gate electrode section 5 and the second gate electrode section 6 and a source-drain voltage V_{sd} is applied between the source electrode 3 and the drain electrode 4, an extremely thin gate insulating film is formed in the ion liquid L, the source-drain current I_{sd} flows to the graphene 8, and the gate insulating film can operate as a transistor. In addition, in the gas sensor 1 having such a configuration, when the ion liquid L absorbs a detection target gas, a state of the gate insulating layer (the electric double layer) in the ion liquid L changes according to an absorption amount of the gas. A source-drain current/gate voltage characteristic can also change according to the state change of the gate insulating layer. Note that, in the gas sensor 1, since the extremely thin electric double layer is formed around the graphene 8, simply by applying, for example, an extremely small gate voltage V_{g} of 1 [V] or less, the source-drain current I_{sd} can flow.

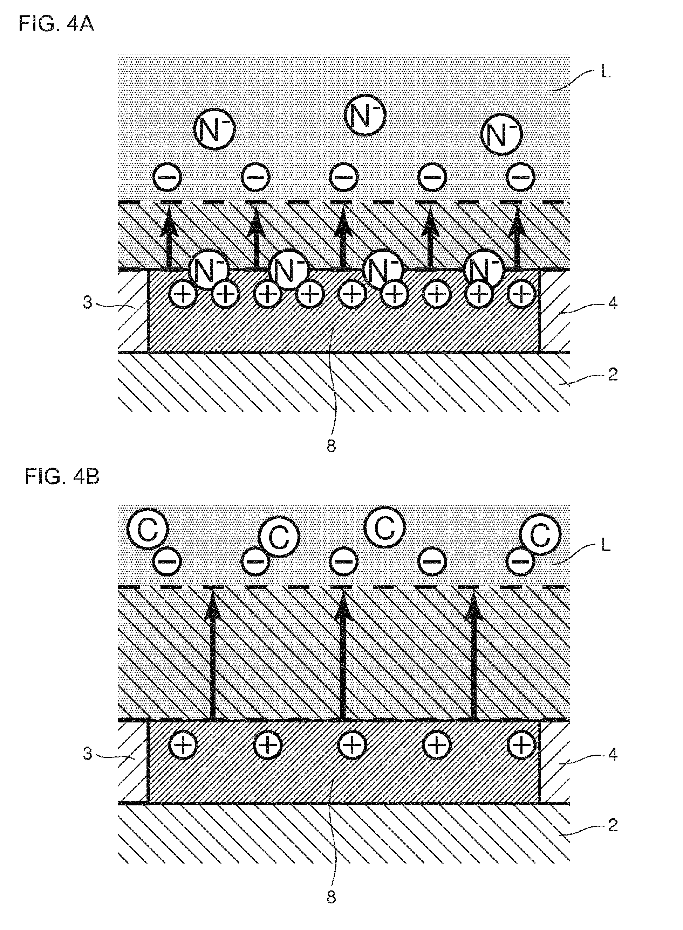

For example, as shown in FIG. 4A, in the gas sensor 1 that uses the ion liquid L capable of absorbing NH₃, when the ion liquid L absorbs NH₃, a state of the negative charges gathering on the surface of the graphene 8 in the ion liquid L changes according to the absorption of NH₃. A state of the electric double layer also changes according to the change in the state of the negative charges and the source-drain current I_{sd} flowing to the graphene 8 changes. As shown in FIG. 4B, similarly, in the gas sensor 1 that uses the ion liquid L capable of absorbing CO₂, when the ion liquid L absorbs CO₂, a state of the negative charges gathering on the surface of the graphene 8 in the ion liquid L changes according to the absorption of CO₂. A state of the electric double layer also changes according to the change in the state of the negative charges and the source-drain current I_{sd} flowing to the graphene 8 also changes.

In this way, by measuring such a change in the source-drain current/gate voltage characteristic, the gas sensor 1 can detect the detection target gas on the basis of the change in the source-drain current/gate voltage characteristic. The gas sensor 1 is configured to be capable of measuring a change amount of the source-drain current/gate voltage characteristic and, when the change amount is large, indicating that gas concentration in gas around the ion liquid L (this is hereinafter simply referred to as outside air) is high and, on the other hand, when the change amount is small, indicating that the gas concentration in the outside air is low, and estimating the gas concentration in the outside air.

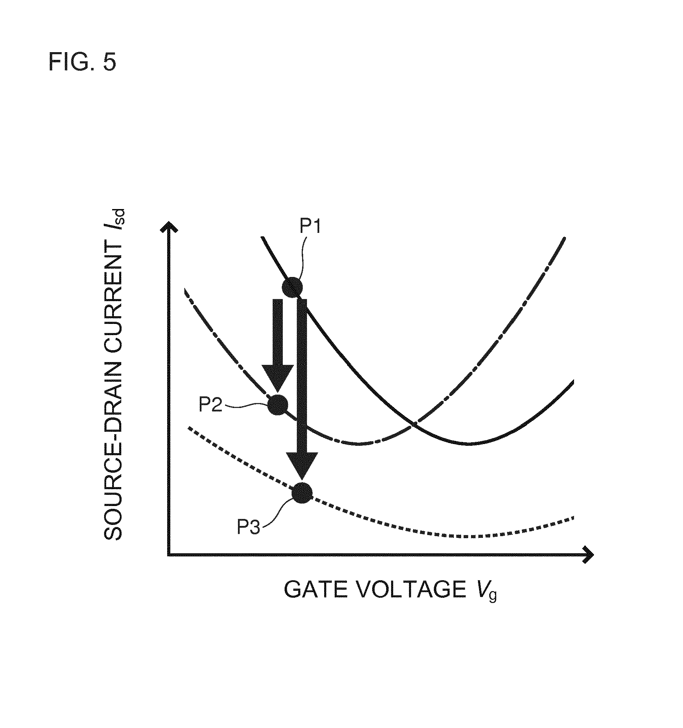

Practically, in the gas sensor 1, as shown in FIG. 5, when the detection target gas is not included in the gas around the ion liquid L, a gentle waveform P1 close to a V shape is obtained as a relation between the source-drain current I_{sd} and the gate voltage V_{g}. On the other hand, when the ion liquid L capable of absorbing NH₃ is used, when the detection target NH₃ is included in the gas around the ion liquid L, a waveform P2 in which the gate voltage V_{g} in the gas sensor 1 shifts in the negative direction is obtained. When the gas concentration in the outside air increases, an amount of the shifted voltage can increase in proportion to the increase in the gas concentration.

On the other hand, when the ion liquid L capable of absorbing CO₂ is used, when the detection target CO₂ is included in the gas around the ion liquid L, a waveform P3 in which the source-drain current I_{sd} in the gas sensor 1 decreases as a whole is obtained. When the gas concentration in the outside air increases, an amount of the decreased source-drain current I_{sd} can increase in proportion to the increase in the gas concentration.

In this way, the gas sensor 1 of the present invention is configured to be capable of detecting gas included in the outside air and estimate a content of the gas on the basis of a change in the source-drain current I_{sd} and a change in the gate voltage V_{g} caused by the change in the source-drain current I_{sd}.

Note that, in the embodiment explained above, the gas sensor 1 is explained that includes the gate electrode 7, applies the gate voltage V_{g} to the first gate electrode section 5 and the second gate electrode section 6 configuring the gate electrode 7, forms the electric double layer on the surface of the graphene 8 in the ion liquid L, and measures a change in the source-drain current I_{sd} flowing to the graphene 8 when a state of the electric double layer changes by absorption of gas by the ion liquid L. However, the present invention is not limited to this. The gas sensor 1 may be a gas sensor that does not include the gate electrode 7 and simply measures a change in the source-drain current I_{sd} flowing to the graphene 8 between the source electrode 3 and the drain electrode 4 when the ion liquid L absorbs gas.

That is, in the gas sensor 1, even if the gate voltage V_{g} is 0 [V], since the graphene 8 having a large number of holes is present in the ion liquid L, the negative charges in the ion liquid L gather on the surface of the graphene 8. Consequently, in the gas sensor 1, when the ion liquid L absorbs the gas, a state of the negative charges and the positive charges in the ion liquid L changes. The source-drain current I_{sd} flowing in the graphene 8 can also change according to the change in the state. Consequently, even if the gate electrode 7 is not provided, the gas sensor 1 can detect gas included in the outside air and estimate a content of the gas from the change in the source-drain current I_{sd} that flows in the graphene 8.

### (2) Manufacturing method for the gas sensor

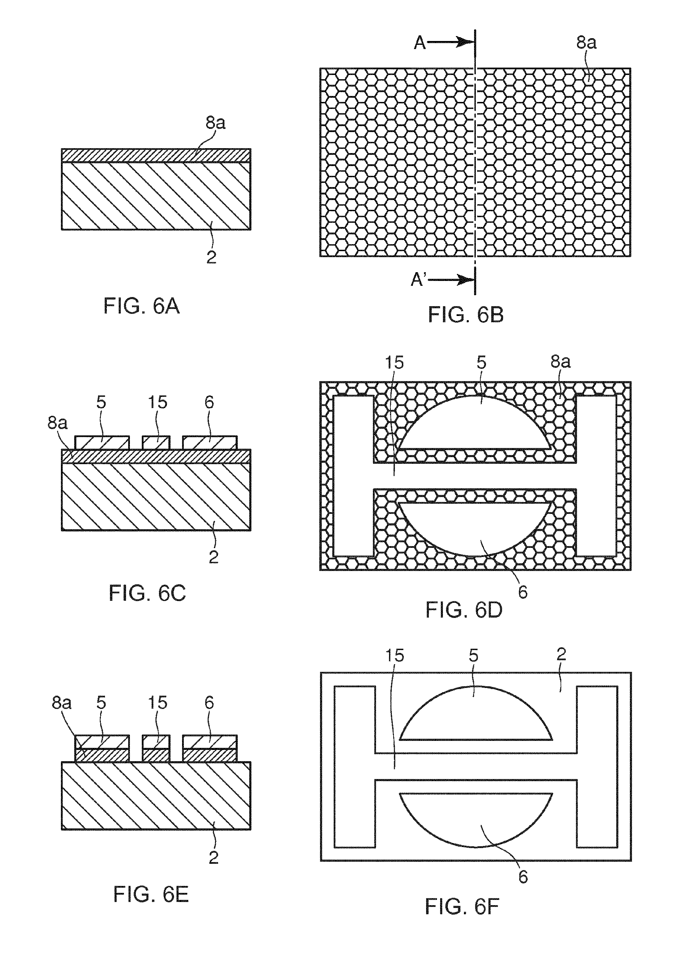

Next, a manufacturing method for the gas sensor 1 of the present invention is explained. In the case of this embodiment, first, for example, the substrate 2 formed of silicon, on the surface of which a thermal oxide film is formed, is prepared. A sheet-like transfer body, on a stamp surface of which a flat graphene layer of a single-layer structure or a plural-layer structure is formed, is prepared. Subsequently, the stamp surface of the transfer body is pressed against the surface of the substrate 2. After the graphene layer is attached to the surface of the substrate 2, only the transfer body is removed. A film-like graphene layer 8a is provided over the entire surface of the substrate 2 as shown in FIG. 6B and FIG. 6A showing a cross section of an A-A' portion of FIG. 6B.

Subsequently, an electrode layer formed of Cr/Au (4[nm]/40[nm]) is formed over the entire surface of the graphene layer 8a. After the electrode layer is patterned using a resist, the resist is removed. As shown in FIG. 6C and FIG. 6D, a source-drain formation electrode section 15, the first gate electrode section 5, and the second gate electrode section 6 are formed on the graphene layer 8a from the electrode layer. Subsequently, as shown in FIG. 6E and FIG. 6F, the graphene layer 8a exposed to the outside in regions other than the source-drain formation electrode section 15, the first gate electrode section 5, and the second gate electrode section 6 is removed by oxygen plasma.

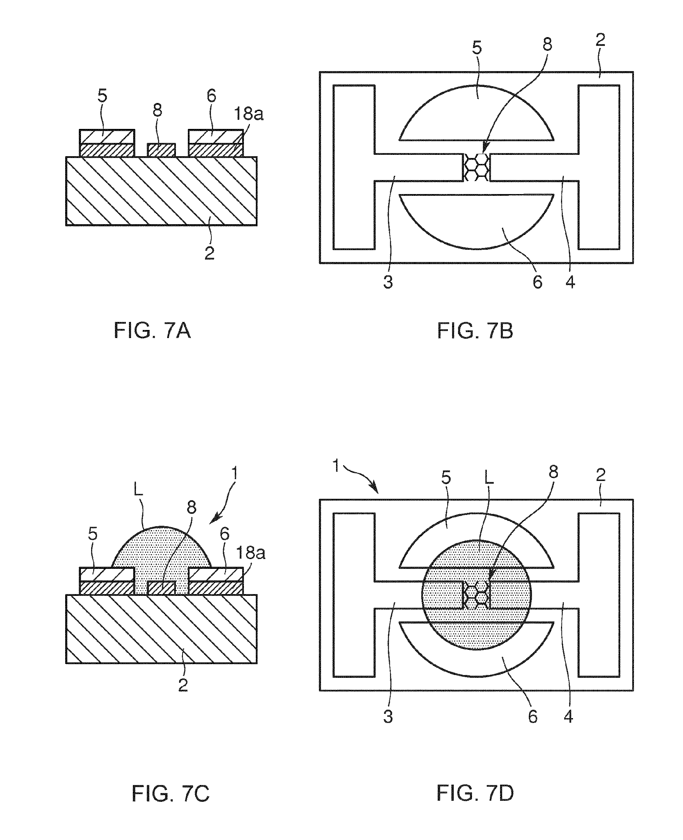

Subsequently, as shown in FIG. 7A and FIG. 7B, a part of the source-drain formation electrode section 15 present between the first gate electrode section 5 and the second gate electrode section 6 is selectively removed by wet etching using an iodine solution to expose a part of the graphene layer 8a to the outside, form the graphene 8 functioning as a channel, and form the source electrode 3 and the drain electrode 4 at both the ends of the graphene 8. Finally, as shown in FIG. 7C and FIG. 7D, the ion liquid L is dropped on the substrate 2 to cover the entire graphene 8 exposed between the source electrode 3 and the drain electrode 4. Consequently, the gas sensor 1 shown in FIG. 1 can be manufactured.

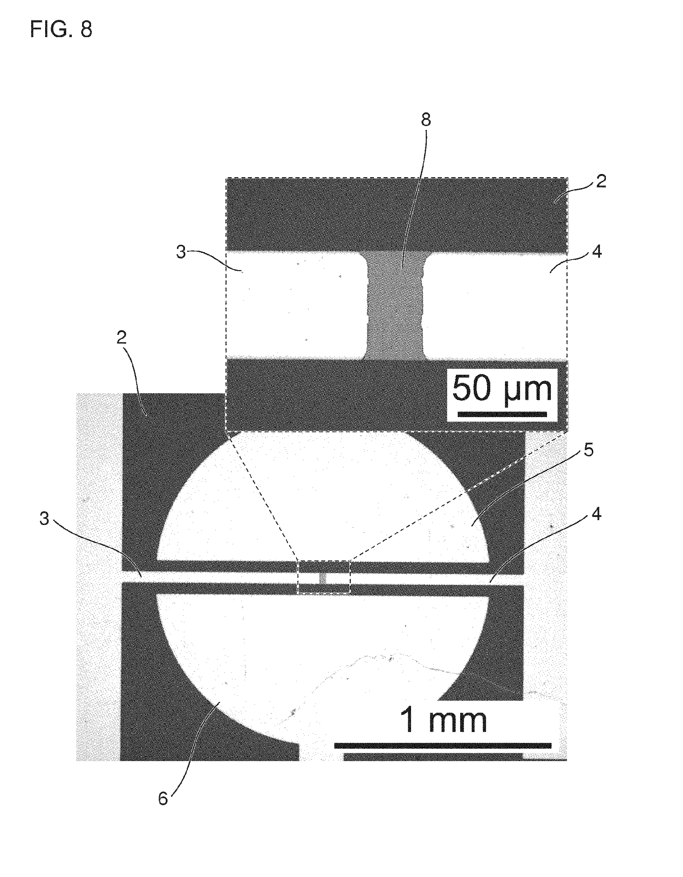

In the gas sensor 1 manufactured in this way, as shown in FIG. 8, the graphene 8 can be formed in a slight gap between the source electrode 3 and the drain electrode 4. The source electrode 3 and the drain electrode 4 can be electrically connected by the graphene 8 formed of an extremely small structure.

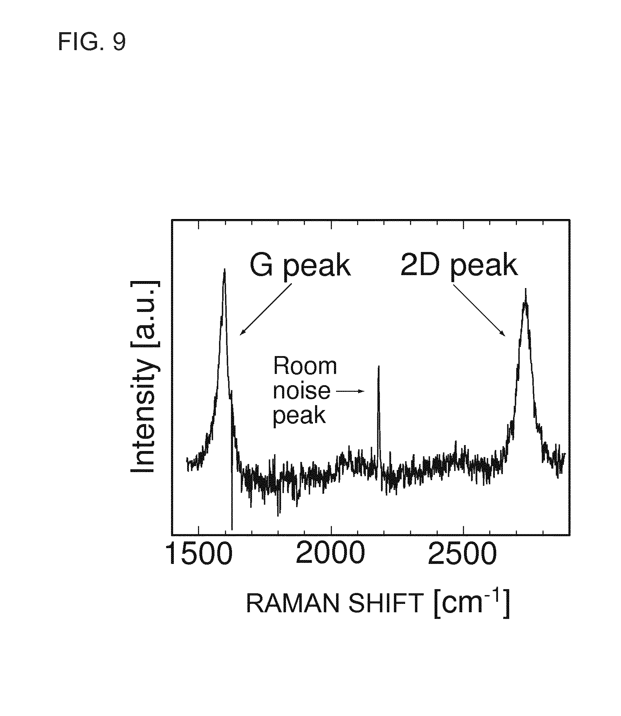

When the graphene 8 was formed on the substrate 2 according to such a manufacturing method using a transfer body in which a graphene layer including approximately three to five mesh-like plane layers was formed, a laser was irradiated on the graphene 8, and a Raman spectrum was analyzed using a Raman spectrometer, a result shown in FIG. 9 was obtained. In the Raman spectrum shown in FIG. 9, a 2D peak at substantially the same height as a G peak was obtained. It is generally known that, when the 2D peak at substantially the same height as the G peak is obtained in this way, a graphene is a graphene including approximately three to five mesh-like plane layers. Therefore, it can be confirmed that, even the graphene 8 finally formed on the substrate 2 according to the manufacturing method is a stacked structure including approximately three to five mesh-like plane layers.

### (3) Verification tests

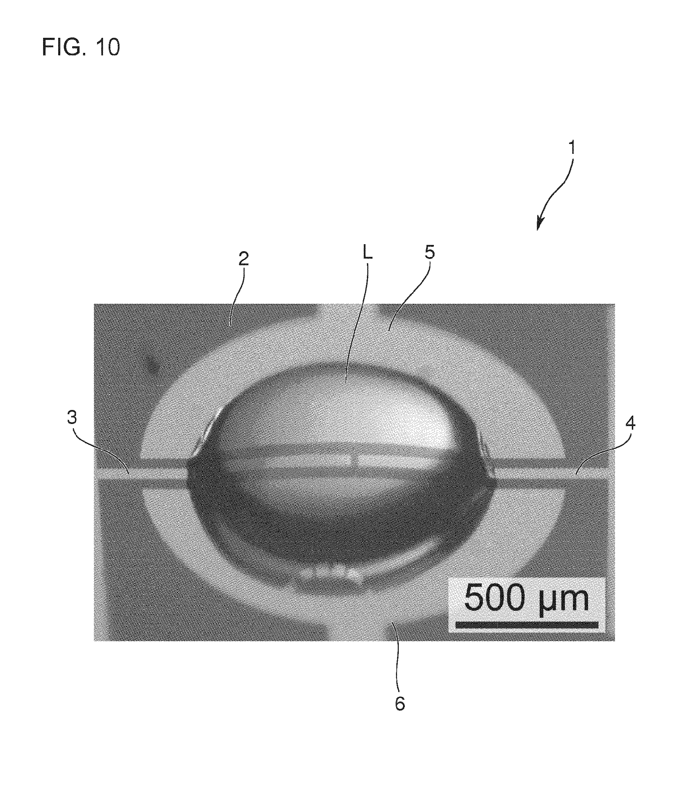

Next, various verification tests are explained. First, each of the source electrode 3, the drain electrode 4, and the gate electrode 7 was formed of Cr/Au (film thickness 4[nm]/40[nm]) according to the manufacturing method explained above. The flat film-like graphene 8 having length of 20 [µm] and width of 50 [µm] was formed between the source electrode 3 and the drain electrode 4 using a transfer body (polydimethylsiloxane, KE-106, Shin-Etsu Chemical Co., Ltd.) in which a graphene layer including approximately three to five mesh-like plane layers was formed and the ion liquid L ([EMIM][BF₄]) was dropped around the graphene 8 to manufacture the gas sensor 1 shown in FIG. 10.

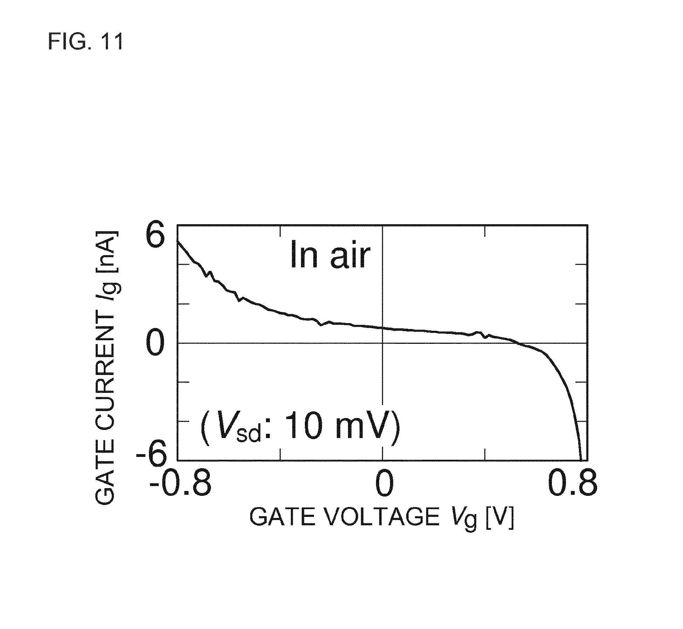

When a detection target gas was not included in the outside air, when a gate current (a leak current) I_{g} (FIG. 2) passing the gate electrode 7 to the drain electrode 4 in the gas sensor 1 was checked, a result shown in FIG. 11 was obtained. It can be confirmed from FIG. 11 that the gate current I_{g} at the source-drain voltage V_{sd} of 10 [mV] is sufficiently small and negligible magnitude and does not affect the measurement of the source-drain current I_{sd}.

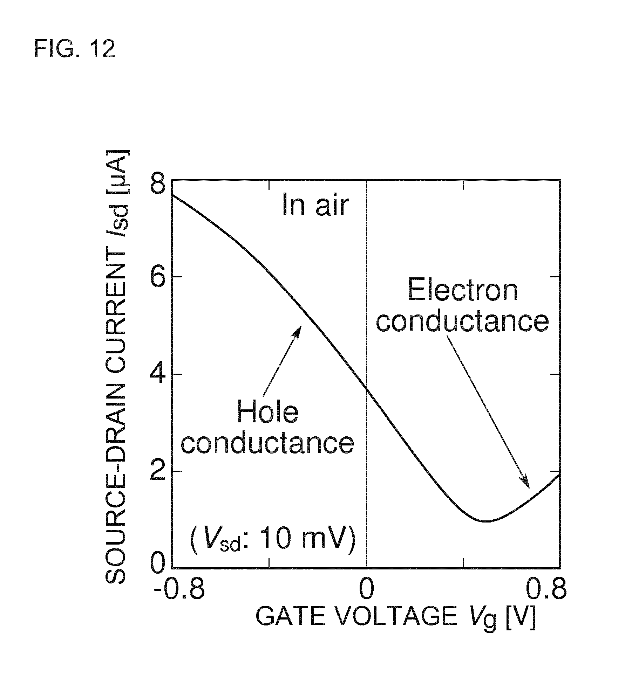

Subsequently, when a relation between the source-drain current I_{sd} flowing from the source electrode 3 to the drain electrode 4 through the graphene 8 and the gate voltage V_{g} applied to the gate electrode 7 was checked, a result shown in FIG. 12 was obtained. Note that FIG. 12 shows a relation between the source-drain current I_{sd} and the gate voltage V_{g} at the time when the source-drain voltage V_{sd} between the source electrode 3 and the drain electrode 4 of the gas sensor 1 was set to 10 [mV] and the gate voltage V_{g} applied to the gate electrode 7 was increased from -0.8 [V] to 0.8 [V]. It can be confirmed from FIG. 12 that, in the gas sensor 1, when the detection target gas is not included in the outside air, a waveform close to a gentle V shape is obtained.

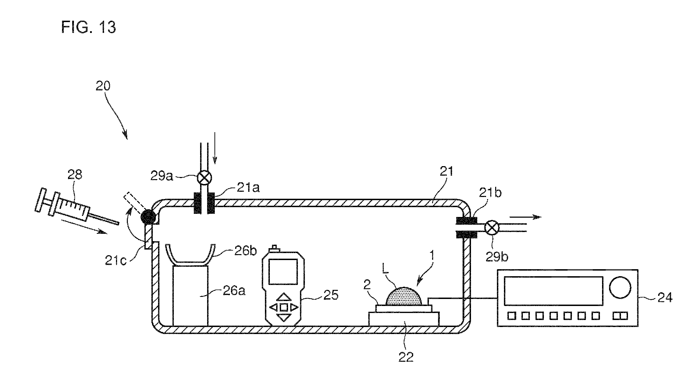

Subsequently, a verification test concerning whether a detection target gas was detectable by the gas sensor 1 was performed using an experimental apparatus 20 shown in FIG. 13. In practice, the experimental apparatus 20 was configured to be capable of supplying the detection target gas from a supply port 21a into a chamber 21 via a valve 29a and configured to be capable of discharging the gas to the outside of the chamber 21 from a discharge port 21b via a valve 29b.

In the chamber 21, an opening/closing door 21c was provided in a wall section and a saucer 26b was provided via a base 26a on the inside near the opening/closing door 21c. An NH₃ solution was able to be supplied to the saucer 26b from the outside using an injector 28 by opening the opening/closing door 21c. In the experimental apparatus 20, a CO₂ measuring device 25 was provided in the chamber 21 to make it possible to measure CO₂ concentration in the chamber 21. In the experimental apparatus 20, the gas sensor 1 of the present invention was placed on the base 22 in the chamber 21. A measuring device 24 provided outside the chamber 21 and the gas sensor 1 in the chamber 21 were connected while maintaining a sealed state of the chamber 21.

In such an experimental apparatus 20, first, after the gas sensor 1 that used 0.1 [µL] of [EMIM][BF₄] as the ion liquid L was set in the chamber 21, the chamber 21 was filled with the air not including a detection target gas. A relation between the source-drain current I_{sd} and the gate voltage V_{g} in the gas sensor 1 at this point was checked. Thereafter, 1 to 150 [µL] of a 28[%] NH₃ solution was supplied from the opening/closing door 21c to the saucer 26b in the chamber 21 using the injector 28. The relation between the source-drain current I_{sd} and the gate voltage V_{g} in the gas sensor 1 was checked at each of NH₃ concentrations 50 [ppm], 500 [ppm], and 5000 [ppm].

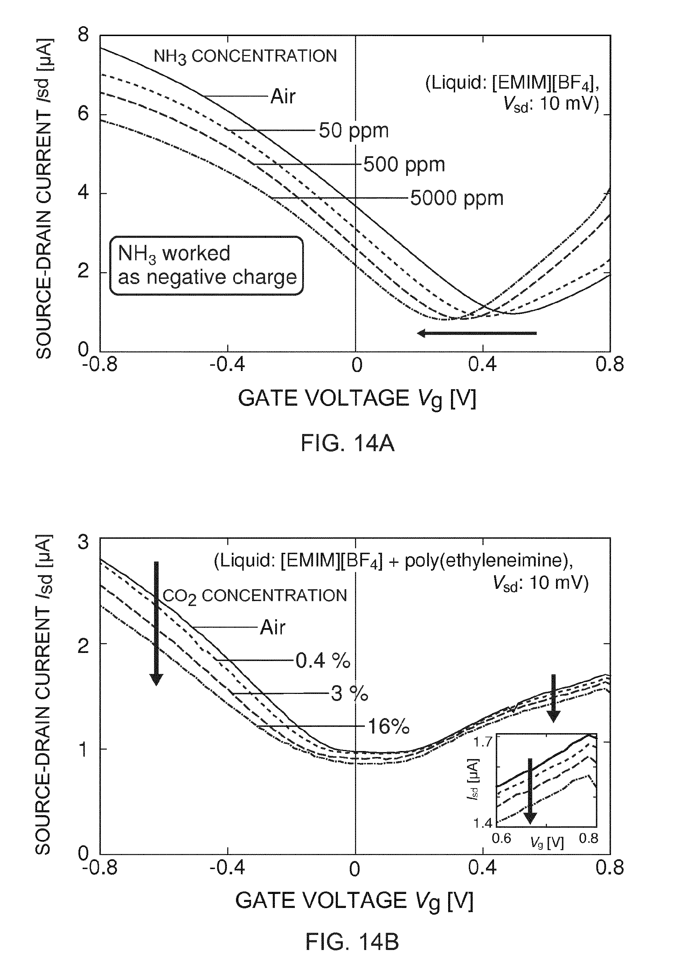

Specifically, when the source-drain voltage V_{sd} was set to 10 [mV], the gate voltage V_{g} applied to the gate electrode 7 was increased from -0.8 [V] to 0.8 [V], and the source-drain current I_{sd} was measured in the gas sensor 1, a result shown in FIG. 14A was obtained. It can be confirmed from FIG. 14A that, in the gas sensor 1 of the present invention, when the detection target NH₃ is mixed in the outside air, the gate voltage V_{g} shifts to the negative side compared with the gate voltage V_{g} in the case of the normal outside air.

In FIG. 14A, the source-drain current I_{sd} at the time when the chamber 21 is filled with the air is not the minimum at the gate voltage V_{g} of 0 [V] and is the minimum at approximately 0.5 [V]. This is estimated to be because a large number of holes are present in the graphene 8. It can be estimated that, when the gate voltage V_{g} is set lower than appropriately 0.5 [V], when NH₃ is absorbed by the ion liquid L, negative charges are given to the hole-rich graphene 8 by NH₃ in the ion liquid L and, since the number of holes in the graphene 8 decreases, a resistance value increase and the source-drain current I_{sd} decreases. Note that it can be estimated that, when the gate voltage V_{g} is set higher than approximate 0.5 [V], conversely, electrons are given to the ion liquid L from the graphene 8 and, when NH₃ is absorbed by the ion liquid L, the source-drain current I_{sd} increases.

Therefore, it can be estimated that, when CO, NO₂, and the like is set as gas detection targets, when the ion liquid L capable of absorbing these gases absorbs the gases, the gas sensor 1 works to deprive negative charges gathered on the surface of the graphene 8 in the ion liquid L and the shift of the source-drain current I_{sd} shown in FIG. 14A changes to a shift to the positive side.

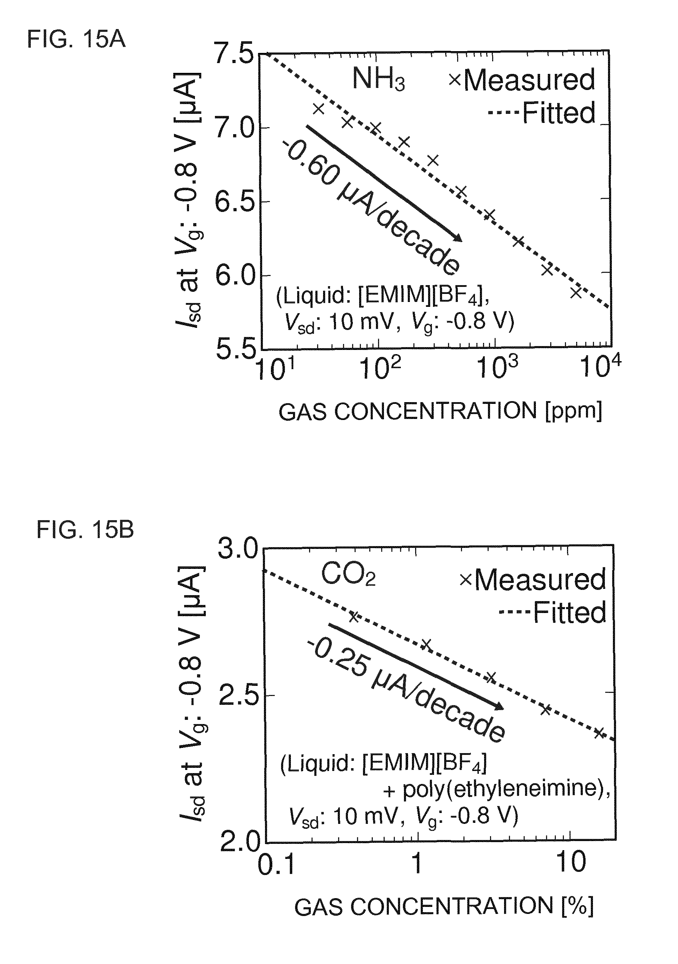

It can be confirmed that a shift amount of the source-drain current I_{sd} is different depending on NH₃ concentration. Specifically, when the gate voltage V_{g} was -0.8 [µm], the NH₃ concentration was further changed, and the source-drain current I_{sd} at each of the NH₃ concentrations was checked, a result shown in FIG. 15A was obtained. In FIG. 15A, measurement points are shown (written as x "Measured"). A change tendency of the source-drain current I_{sd} from the measurement points was checked (written as "Fitted"). As a result, it can be confirmed that, in the gas sensor 1, the source-drain current I_{sd} decreases -0.60 [µA] every time the NH₃ concentration changes by ten times and the source-drain current I_{sd} decreases in proportion to the NH₃ concentration change. Therefore, in the gas sensor 1 of the present invention, it is possible to estimate the NH₃ concentration from the source-drain current I_{sd} by causing the measurement device to store data obtained by checking a correspondence relation between the NH₃ concentration and the source-drain current I_{sd} in advance.

From the above, it can be confirmed that, in the gas sensor 1, when the ion liquid L absorbs NH₃ as the detection target, the source-drain current I_{sd} flowing to the graphene 8 changes and a shift voltage occurs. Consequently, it can be confirmed that the gas sensor 1 of the present invention can detect NH₃ in the outside air around the ion liquid L by measuring a change in the source-drain current I_{sd}. It can be confirmed that the gas sensor 1 of the present invention can also determine the NH₃ concentration on the basis of the shift amount of the source-drain current I_{sd}.

Subsequently, after the inside of the chamber 21 was refreshed, the detection target was changed from NH₃ to CO₂. A new experiment was separately performed under experiment conditions same as the above. In the experiment, in order to cause the ion liquid L to absorb CO₂, the gas sensor 1 that uses the ion liquid L added with a small amount of polyethyleneimine (PEI) in [EMIM][BF₄] was set in the chamber 21. Subsequently, CO₂ was supplied into the chamber 21 via the supply port 21a, CO₂ was mixed in the outside air (the air), and the chamber 21 was sequentially filled with a mixed gas. When CO₂ concentration stabilized as each of 0.4[%], 3[%], and 16[%], a relation between the source-drain current I_{sd} and the gate voltage V_{g} in the gas sensor 1 was checked.

Note that, in this case as well, the source-drain voltage V_{sd} was set to 10 [mV], the gate voltage V_{g} applied to the gate electrode 7 was increased from -0.8 [V] to 0.8 [V], and the source-drain current I_{sd} was measured in the gas sensor 1. As a result, a result shown in FIG. 14B was obtained. It can be confirmed from FIG. 14B that, in the gas sensor 1 of the present invention, when the detection target CO₂ is mixed in the outside air, the source-drain current I_{sd} decreases as a whole within the gate voltage V_{g} compared with the source-drain current I_{sd} in the case of the normal outside air.

In FIG. 14B, a waveform is not the waveform shown in FIG. 14A in which the source-drain current I_{sd} shifts. As shown in FIG. 14B, when a value of the source-drain current I_{sd} decreases as a whole within all of the gate voltages V_{g}, this is because the resistance of the graphene 8 itself decreases or because of a change in the thickness of the electric double layer. However, since a change of a voltage value is approximately 10[%] in FIG. 14B, it is hard to consider that the decrease in the value of the source-drain current I_{sd} is caused by a resistance change of the graphene 8 itself. It is estimated that the decrease in the value of the source-drain current I_{sd} is caused by the thickness change of the electric double layer. Therefore, in this case, as shown in FIG. 4B, it is seen that the electric double layer increases in thickness and the source-drain current I_{sd} decreases according to the contribution of the thickness change of the electric double layer.

It can also be confirmed that the source-drain current I_{sd} decreases as the CO₂ concentration increases. Specifically, when the gate voltage V_{g} was -0.8 [V], the CO₂ concentration was further changed, and the source-drain current I_{sd} at each of the CO₂ concentrations was checked, a result shown in FIG. 15B was obtained. In FIG. 15B, measurement points are shown (written as x "Measured"). A change tendency of the source-drain current I_{sd} from the measurement points was checked (written as "Fitted"). As a result, in this example, it can be confirmed that the source-drain current I_{sd} decreases -0.25 [µA] every time the CO₂ concentration changes by ten times and the source-drain current I_{sd} decreases in proportion to the CO₂ concentration change. Therefore, in the gas sensor 1 of the present invention, it is possible to estimate the CO₂ concentration from the source-drain current I_{sd} by causing the measurement device to store data obtained by checking a correspondence relation between the CO₂ concentration and the source-drain current I_{sd} in advance.

Therefore, it can be confirmed that, in the gas sensor 1, the ion liquid L absorbs CO₂ as the detection target and, as a result, the source-drain current I_{sd} flowing to the graphene 8 decreases. Consequently, it can be confirmed that the gas sensor 1 of the present invention can detect CO₂ in the outside air around the ion liquid L by measuring a change in the source-drain current I_{sd}. It can be confirmed that the gas sensor 1 of the present invention can also determine the CO₂ concentration on the basis of a decrease amount of the source-drain current I_{sd}.

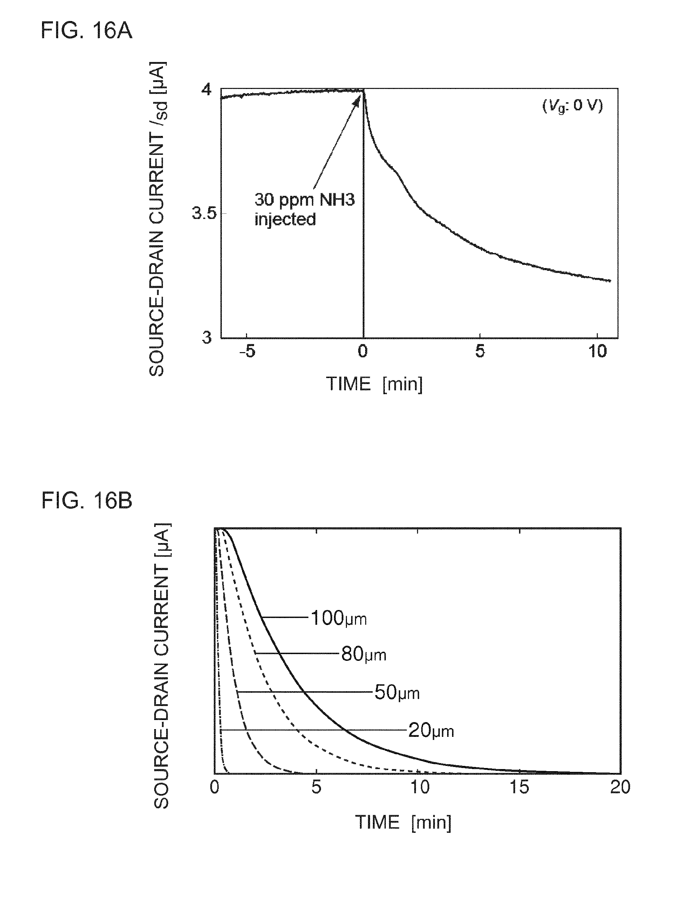

Subsequently, when it was checked after the elapse of how long time the source-drain current I_{sd} started to change when the gas sensor 1 having the gate voltage V_{g} of 0 [V] was set under an NH₃ atmosphere, a result shown in FIG. 16A was obtained. Note that, in this verification experiment, the NH₃ concentration was set to 30 [ppm] and, in the gas sensor 1, the distance between the surface of the ion liquid L including [EMIM][BF₄] and the graphene 8 was set to 80 [µm]. It can be confirmed from FIG. 16A, in the gas sensor 1, the source-drain current I_{sd} stabilizes when approximately 10 minutes elapses after the gas sensor 1 is set under the NH₃ atmosphere.

When a theoretical formula was calculated from a result of such a verification experiment and response times of the source-drain current I_{sd} at the time when the distance between the graphene 8 and the surface of the ion liquid L including [EMIM][BF₄] was set to 20 [µm], 50 [µm], 80 [µm], and 100 [µm] were simulated, a result shown in FIG. 16B was obtained. It can be confirmed from FIG. 16B that time until the source-drain current I_{sd} stabilizes in the gas sensor 1 decreases when the distance between the graphene 8 and the ion liquid L surface is reduced. This is considered to be because it takes time until gas absorbed from the surface of the ion liquid L diffuses to the vicinity of the graphene 8. Therefore, in the present invention, it can be confirmed that it is possible to realize a gas sensor capable of detecting a detection target gas early by using the gas sensor 1 in which the distance between the graphene 8 and the ion liquid L surface is set short.

### (4) Action and effects

In the configuration explained above, in the gas sensor 1, the graphene 8 is provided between the source electrode 3 and the drain electrode 4 on the substrate 2 and the graphene 8 is covered by the ion liquid L. In such a gas sensor 1, since the graphene 8 having a large number of holes is present in the ion liquid L, the negative charges in the ion liquid L gather on the surface of the graphene 8. Consequently, in the gas sensor 1, when the ion liquid L absorbs the detection target gas, a state of the negative charges gathered on the surface of the graphene 8 in the ion liquid L changes. The source-drain current I_{sd} flowing to the graphene 8 also changes according to the change in the state. Therefore, it is possible to detect gas in the outside air on the basis of a tendency of the change in the source-drain current I_{sd}.

In the case of this embodiment, in the gas sensor 1, the ion liquid L functioning as the gate insulating layer is provided in contact with the graphene 8 and the gate electrode 7 on the substrate 2. The gate voltage V_{g} is applied to the ion liquid L via the gate electrode 7. Consequently, in the gas sensor 1, the electric double layer functioning as the gate insulating layer is formed in the ion liquid L that absorbs gas. The electric double layer can be operated as a transistor capable of measuring the source-drain current I_{sd} flowing to the graphene 8.

In this case, in the gas sensor 1, when the ion liquid L absorbs the detection target gas, a state of the gate insulating layer (the electric double layer) in the ion liquid L changes. The source-drain current I_{sd} flowing to the graphene 8 changes according to the state of the gate insulating layer. Therefore, by measuring the change in the source-drain current I_{sd}, it is possible to detect gas in the outside air on the basis of a tendency of the change in the source-drain current I_{sd}.

In the conventional gas sensor (not shown in the figure) of the back gate type described in National Publication of International Patent Application No. 2007-505323, a silicon oxide film having film thickness of, for example, 150 to 200 [nm] is used as a gate insulating film between the silicon back gate and the carbon nanotubes. Therefore, a gate voltage of approximately maximum 15 [V] is necessary to operate the gate insulating layer as a transistor.

On the other hand, in the gas sensor 1 of the present invention, an extremely thin gate insulating layer of several nanometers is formed in the ion liquid L provided between the graphene 8 and the gate electrode 7 without using a silicon oxide film of SiO₂ or the like. Therefore, even if the gate voltage V_{g} of, for example, approximately 0.4 [V] is applied to the gate electrode 7, the gate insulating layer can operate as the transistor. It is possible to more markedly reduce the gate voltage V_{g} than in the conventional gas sensors.

In the gas sensor 1, the gate insulating layer is formed in the ion liquid L itself that absorbs gas. A state change of the gate insulating layer of the ion liquid L caused by the absorption of the gas is directly reflected on the source-drain current I_{sd} that flows in the graphene 8. Therefore, it is possible to improve detection sensitivity of the gas more than in the conventional gas sensors. Further, in the gas sensor 1, unlike in the conventional gas sensors, it is unnecessary to apply the surface chemical modification to the graphene 8 itself. The ion liquid L only has to be provided in contact with the graphene 8 and the gate electrode 7. Therefore, it is possible to simplify a configuration.

In the gas sensor 1, a source-drain current/gate voltage characteristic changes according to gas concentration in the outside air. Therefore, by measuring a change amount of the source-drain current/gate voltage characteristic, it is possible to estimate on the basis of the change amount to which degree a detection target gas is included in the outside air.

Incidentally, as such a gas sensor, a gas sensor having a configuration in which carbon nanotubes are provided along a substrate surface between a source electrode and a drain electrode on a substrate and the carbon nanotubes are covered with ion liquid (hereinafter referred to as carbon nanotube type gas sensor) is also conceivable. However, when the carbon nanotubes are formed on the substrate, a part of a side circumferential surface is disposed to be in line-contact with the substrate. Therefore, a region where the part of the side circumferential surface is in line-contact with the substrate is in a non-contact state with the ion liquid. Therefore, there is a problem in that a state change of the ion liquid that absorbs gas is less easily reflected. The carbon nanotubes are in line-contact on the substrate. Therefore, there is also a problem in that a fixing area with respect to the substrate is extremely small and the carbon nanotubes easily peel from the substrate with static electricity or the like generated in a manufacturing process and are easily damaged.

On the other hand, in the gas sensor 1 of the present invention, the entire surface of the flat film-like graphene 8 is formed in surface-contact on the substrate 2. The entire surface of the graphene 8 is configured to be exposed in the ion liquid L. Consequently, in the gas sensor 1 of the present invention, a region in which the graphene 8 is in a non-contact state with the ion liquid L is smaller than the region in the conventional gas sensors. Therefore, a state change of the ion liquid L that absorbs gas is easily reflected on a change in the source-drain current I_{sd} that flows in the graphene 8. It is possible to improve responsiveness of gas detection. In particular, when the graphene 8 is formed of a single-layer structure, substantially all carbon atoms can be exposed in the ion liquid L. Therefore, it is possible to further improve the responsiveness of gas detection. In the present invention, since the entire flat surface of the graphene 8 is in surface-contact with the substrate 2, a fixing area to the substrate is also large. Even if static electricity or the like occurs in a manufacturing process, the graphene 8 less easily peels from the substrate 2. It is possible to prevent damage in the manufacturing process.

When the conventional carbon nanotube type gas sensor is manufactured, a catalyst section is provided on the substrate and carbon is grown from the catalyst section by chemical vapor deposition (CVD) to form carbon nanotubes linearly extending from the catalyst section on the substrate. However, the carbon nanotubes manufactured in this way do not always linearly extend from the catalyst section. It is also highly likely that a defective manufactured product extending in a curved shape from the catalysis section is manufactured. The diameter of the carbon nanotubes and a disposition state of a six-membered ring structure on a cylindrical surface easily change depending on conditions of the manufacturing process. There is a problem in that it is difficult to manufacture carbon nanotubes having the same characteristic in mass production.

On the other hand, in the gas sensor 1 according to the present invention, in manufacturing, the graphene layer 8a formed on the surface of the transfer body in advance is only attached to the surface of the substrate 2. Therefore, a defective manufactured product is not manufactured. It is possible to surely use the graphene 8 having uniform characteristics. Therefore, it is possible to keep gas detection abilities of all gas sensors 1 uniform. The gas sensor 1 is more excellent in mass production than in the conventional gas sensors.

With the configuration explained above, the graphene 8 between the source electrode 3 and the drain electrode 4 is provided in the ion liquid L. Consequently, a state change of charges in the ion liquid L caused by absorption of gas is directly reflected on the source-drain current I_{sd} that flows in the graphene 8. Therefore, it is possible to further improve detection sensitivity of gas more than in the conventional gas sensors. The graphene 8 only has to be disposed in the ion liquid L. Therefore, unlike in the conventional gas sensors, it is unnecessary to apply the surface chemical modification to the carbon nanotubes with a plurality of polymers. Therefore, it is possible to simplify a configuration.

### (5) Other embodiments

Note that the present invention is not limited to this embodiment. Various modified implementations are possible within the range of the gist of the present invention. In the embodiment explained above, the entire flat surface of the graphene 8 functioning as the carbon structural body is disposed in surface-contact with the surface of the substrate 2. However, the present invention is not limited to this. For example, a carbon structural body formed of any one of a graphene, a graphene stacked body, and graphite may be disposed upright on the substrate 2 to dispose a flat surface of the carbon structural body perpendicularly to the surface of the substrate 2.

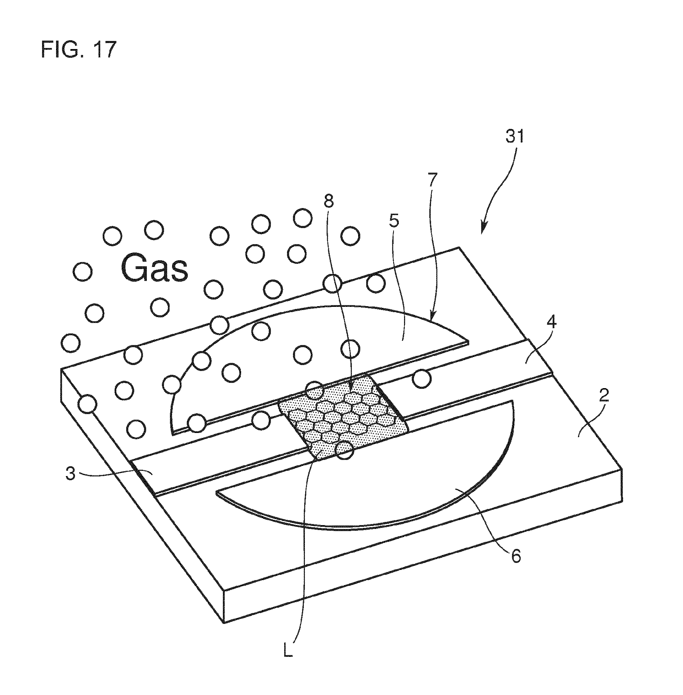

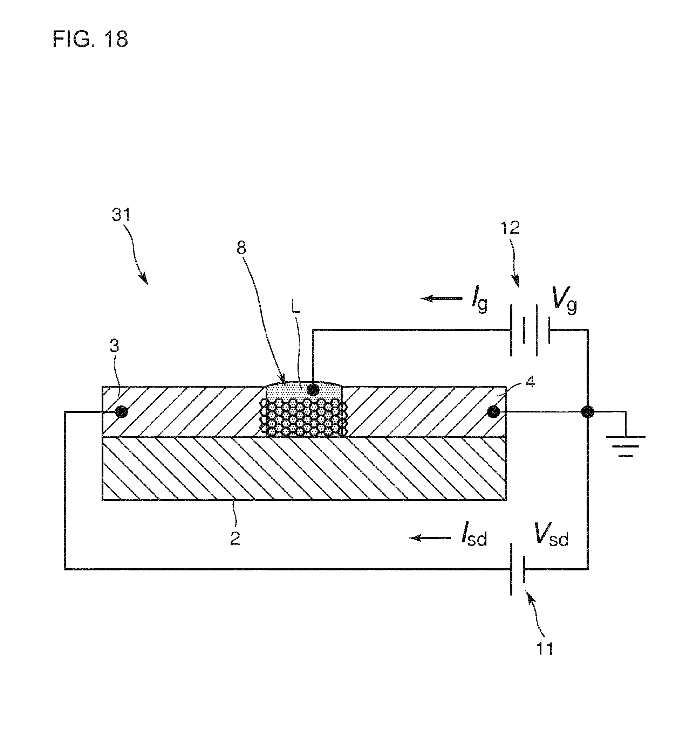

In the embodiment explained above, in the gas sensor 1, the ion liquid L is provided to be placed not only on the source electrode 3 and the drain electrode 4 but also on the first gate electrode section 5 and the second gate electrode section 6. However, the present invention is not limited to this. As shown in FIG. 17 in which portions corresponding to the portions shown in FIG. 1 are denoted by the same reference and FIG. 18 showing a sectional configuration of FIG. 17, a gas sensor 31 may be applied in which the ion liquid L is provided, without covering the upper surfaces of the source electrode 3, the drain electrode 4, the first gate electrode section 5, and the second gate electrode section 6, only in a region surrounded by the source electrode 3, the drain electrode 4, the first gate electrode section 5, and the second gate electrode section 6.

Practically, in the gas sensor 31, the ion liquid L is disposed in a region surrounded by the source electrode 3, the drain electrode 4, the first gate electrode section 5, and the second gate electrode section 6 to be in contact with the side surfaces of the source electrode 3, the drain electrode 4, the first gate electrode section 5, and the second gate electrode section 6. Therefore, it is possible to stably provide the ion liquid L on the substrate 2 with the action of surface tension while reducing an amount of the ion liquid L and attaining a reduction in size. In the gas sensor 31, as in the embodiment explained above, when the gate voltage is applied to the ion liquid L via the gate electrode 7, an electric double layer of several nanometers functioning as a gate insulating layer is formed in the ion liquid L. It is possible to reduce the ion liquid L to such volume with which the gate insulating layer of approximately several nanometers can be formed.

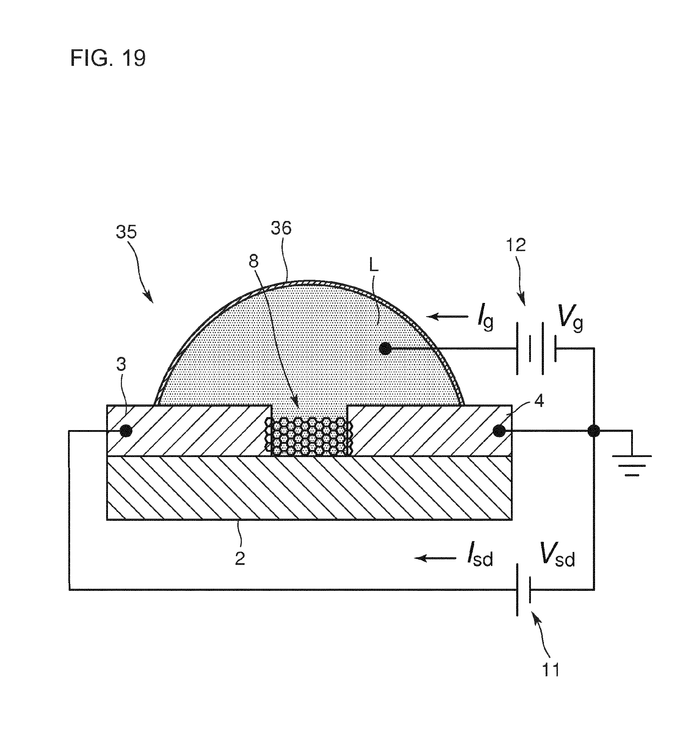

In the embodiment explained above, in the gas sensor 1, the ion liquid L is simply dropped and placed on the substrate 2. However, the present invention is not limited to this. As another embodiment, as shown in FIG. 19 in which portions corresponding to the portions shown in FIG. 2 are denoted by the same reference numerals and signs, a gas sensor 35 may be applied that has a configuration in which a liquid surface formed in a curved shape of the ion liquid L is covered by a coating film 36 such as parylene through which the outside air can pass. In this case, even if, for example, external force is applied to the substrate 2 and the substrate 2 tilts, it is possible to continue to stably retain the ion liquid L on the substrate 2 with the coating film 36 functioning as a retainer. Incidentally, the gas sensor 35 can be manufactured by, after dropping the ion liquid L, forming a coating material such as parylene, through which the outside air can pass, on the ion liquid L by, for example, a CVD (Chemical Vapor Deposition) method, and forming the coating film 36 directly on the ion liquid L. The gas sensor 35 can also be manufactured by forming the coating film 36 on the substrate 2 in advance with the coating material such as parylene, through which the outside air can pass, injecting the ion liquid L into the coating film 36, thereby sealing the ion liquid L with the coating film 36.

In such a gas sensor 35, the ion liquid L can be stably retained on the substrate 2 by the coating film 36. Therefore, for example, it is also possible to set the substrate 2 on a ceiling in a room in a state in which the ion liquid L is directed downward. It is possible to set the ion liquid L to be directed in various directions according to situations of use. In such a gas sensor 35, since a gas absorbent can be isolated from the outside air, it is also possible to use volatile liquid such as water as the gas absorbent. Note that, even if the water is used as the gas absorbent, according to a state change of charges in the water by absorption of gas by the water, the source-drain current I_{sd} flowing in the graphene 8 changes. It is possible to attain effects same as the effects in the embodiment explained above.

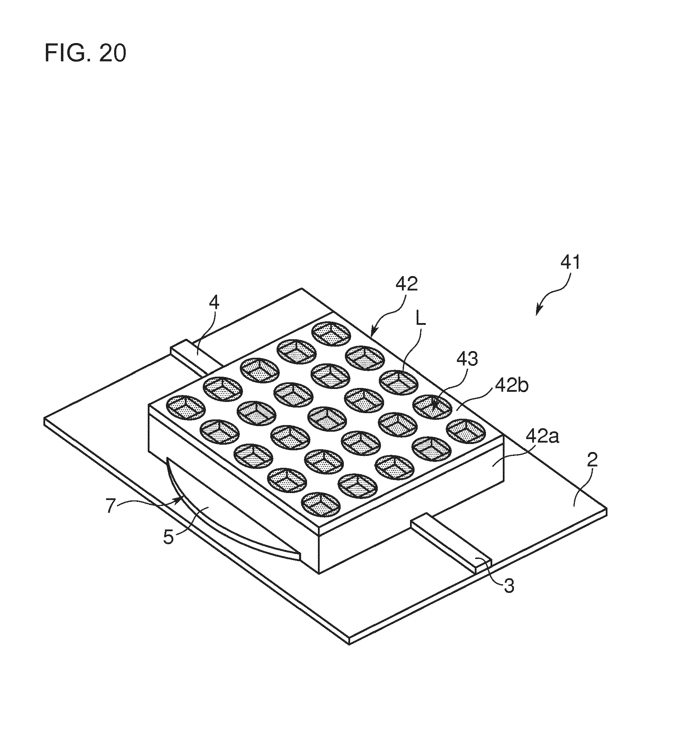

As a gas sensor according to another embodiment, as shown in FIG. 20 in which portions corresponding to the portions shown in FIG. 1 are denoted by the same reference numerals and signs, a gas sensor 41 can also be applied in which a frame body 42 that covers the ion liquid L is provided on the substrate 2 and the ion liquid L is retained on the substrate 2 by the frame body 42 functioning as a retainer. In this case, the frame body 42 is disposed on the substrate 2 to cover the graphene 8 (not shown in the figure) between the source electrode 3 and the drain electrode 4 on the substrate 2 and cover a part of the source electrode 3, the drain electrode 4, and the gate electrode 7 disposed around the graphene 8. The frame body 42 is configured to be capable of retaining the ion liquid L in an internal space thereof.

Practically, in the frame body 42, the internal space is formed by a wall section 42a formed in, for example, a quadrilateral shape that dams the ion liquid L and a tabular top plate section 42b disposed to cover the wall section 42a. A plurality of fine through-holes 43 that allow the internal space and the outside to communicate with each other are drilled in the top plate section 42b. When the frame body 42 is set on the substrate 2, a bottom surface section of the frame body 42 is closed. The internal space communicates with the outside only through the through-holes 43. The ion liquid L is injected into the internal space from the through-holes 43, whereby the ion liquid L can be retained in the internal space. In the frame body 42, since the through-holes 43 are fine, the surface tension of the ion liquid L acts in the through-holes 43. The ion liquid L injected into the internal space less easily flows out to the outside from the through-holes 43. The frame body 42 is configured to be capable of surely retaining the ion liquid L.

The ion liquid L covers the carbon structural body in the internal space of the frame body 42 and is in contact with the first gate electrode section 5 and the second gate electrode section 6 (not shown in the figure) of the gate electrode 7. Consequently, in the gas sensor 41 as well, when the gate voltage V_{g} is applied to the ion liquid L from the gate electrode 7, an electric double layer functioning as a gate insulating layer is formed in the ion liquid L. Therefore, it is possible to attain effects same as the effects in the embodiment explained above.

Further, as another embodiment, a configuration may be adopted in which the substrate 2, on which the graphene 8, the gate electrode 7, and the like are provided, is set in a box-like reservoir section in which the ion liquid L is stored and the substrate 2 is immersed in the ion liquid L. Gas sensors having various configurations in which a disposition relation between the substrate 2 and the ion liquid L is changed as appropriate may be applied according to situations of use. The frame body functioning as the retainer may be a mesh-like frame body knitted by thin wires. Further, the coating film functioning as the retainer may be a fiber-like film. In all the cases, the ion liquid L can be retained in the internal space of the retainer.

Further, in the embodiment explained above, the ion liquid L in a liquid state is applied as the gas absorbent. However, the present invention is not limited to this. For example, a gas absorbent formed in a gel state having viscosity and lacking fluidity, a gas absorbent formed in a cream state having viscosity and also having fluidity, and other gas absorbents in semisolid semi-liquid states between a solid state and a liquid state may be applied. In the gas sensors 1, 31, 35, and 41 explained above, for example, an ion gel body having physical properties same as physical properties of the ion liquid L and obtained by changing the ion liquid L to the gel state and an ionic cream body obtained by changing the ion liquid L to the cream state may be applied. For example, in the gas sensor that uses such a gel-like gas absorbent, even if the substrate 2 is tilted or vibrated, the gas absorbent does not flow. It is possible to continue to maintain a state in which the entire graphene 8 is covered with the gas absorbent. Consequently, it is possible to improve a degree of freedom of a setting place of the gas sensor.

In such a gas sensor, when the gel-like gas absorbent lacking fluidity is used, it is possible to provide an adhesive member on the bottom of the gas absorbent. Consequently, it is also possible to fixedly attach the gas absorbent to the substrate 2 with the adhesive member. Consequently, in the gas sensor, even if the gas sensor is vertically reversed to set the substrate 2 on the ceiling or the like and the gas absorbent is disposed above in a state in which the gas absorbent is exposed to the outside, it is possible to maintain a state in which the gas absorbent is fixedly attached to the substrate 2. Therefore, it is possible to continue to maintain the state in which the entire graphene 8 is covered with the gas absorbent. Consequently, it is possible to further improve the degree of freedom of the setting place of the gas sensor. Note that, on the bottom of the gas absorbent, by providing the adhesive member in a frame portion avoiding a contact place with the graphene 8, it is possible to avoid the influence of the adhesive member on the graphene 8.

Further, for example, a gas absorbent formed of a fiber-like substance impregnated with the ion liquid L may be applied. The graphene 8 may be covered with the gas absorbent. A configuration obtained by combining the various configurations explained above may be adopted.

A gas sensor structural body obtained by arraying the gas sensors 1, 31, 35, and 41 having the same configuration among the gas sensors 1, 31, 35, and 41 of the present invention explained above or a gas sensor structural body obtained by combining and arraying a plurality of kinds of the gas sensors 1, 31, 35, and 41 having different configurations may be adopted. In this case, the gas structural body has, for example, a configuration in which a plurality of gas sensors (31, 35, and 41) are arrayed on a substrate. Consequently, the gas sensors 1 (31, 35, and 41) can detect predetermined gases, respectively. One small gas sensor structural body can specify presence or absence of a plurality of different kinds of gases. In such a gas sensor structural body, by using different ion liquid (gas absorber) for each of the gas sensors 1 (31, 35, and 41) and changing responsiveness of the ion liquid bodies and gases, it is possible to comprehensively discriminate specific gas from patterns of responses of the plurality of gas sensors 1 (31, 35, and 41) and detect the concentration of the gas. Consequently, it is possible to detect presence or absence and concentrations of a plurality of different gases with one small gas sensor structural body.

### Reference Signs List

- 1, 31, 35, 41: Gas sensor(s)
- 2: Substrate
- 3: Source electrode
- 4: Drain electrode
- 5: First gate electrode section
- 6: Second gate electrode section
- 7: Gate electrode
- 8: Graphene (Carbon structural body)
- L: Ion liquid (Gas absorber)
- 36: Coating film (Retainer)
- 42: Frame body (Retainer)

## Claims

1. A gas sensor that detects a gas to be targeted for detection, the gas sensor comprising:
a carbon structural body provided between a source electrode and a drain electrode on a substrate, flat and formed of a single-layer structure having thickness equivalent to one carbon atom or a plural-layer structure; and
a gas absorbent disposed to cover the carbon structural body, wherein
the gas sensor detects the gas on the basis of a change in a source-drain current caused in the carbon structural body by absorption of the gas via the gas absorbent.

2. The gas sensor according to claim 1, wherein the gas absorbent is formed in any of a liquid state, a gel state, and a cream state.

3. The gas sensor according to claim 1 or 2, wherein the carbon structural body is a graphene.

4. The gas sensor according to any one of claims 1 to 3, wherein the gas absorbent is in contact with the carbon structural body and a gate electrode on the substrate and functions as a gate insulating layer, a state of the gate insulating layer changes when the gate insulating layer absorbs the gas, and the gas sensor detects the gas on the basis of a change in the source-drain current caused according to the state of the gate insulating layer.

5. The gas sensor according to claim 4, wherein
the gate electrode is configured from a first gate electrode section and a second gate electrode section, and
the carbon structural body is disposed between the first gate electrode section and the second gate electrode section, and the gas absorbent is disposed in contact with the first gate electrode section and the second gate electrode section.

6. The gas sensor according to claim 4, wherein the gas absorbent is retained in a gap formed between the first gate electrode section and the second gate electrode section.

7. The gas sensor according to any one of claims 4 to 6, wherein the gas sensor detects the gas on the basis of a change in a gate voltage of the gate electrode that changes according to the source-drain current.

8. The gas sensor according to any one of claims 1 to 7, wherein a retainer for covering the gas absorbent and retaining the gas absorbent on the substrate is provided.

9. The gas sensor according to any one of claims 1 to 8, wherein the gas absorbent is an ion liquid, an ion gel body, or an ionic cream body.

10. A gas sensor structural body having a configuration in which a plurality of gas sensors are disposed, wherein
the gas sensors are the gas sensor according to any one of claims 1 to 9.

11. The gas sensor structural body according to claim 10, wherein a different kind of gas absorbent is provided for each of the gas sensors.
